Europäisches Patentamt

European Patent Office

Office européen des brevets

(19)

(11) **EP 0 488 888 B1**

(12)

# FASCICULE DE BREVET EUROPEEN

(45) Date de publication et mention
de la délivrance du brevet:
**08.07.1998 Bulletin 1998/28**

(51) Int Cl.⁶: **G06T 11/00**

(21) Numéro de dépôt: **91403213.1**

(22) Date de dépôt: **27.11.1991**

(54) **Procédé et dispositif de reconstruction d'images tridimensionnelles d'un objet en utilisant deux trajectoires circulaires d'axe commun**

Verfahren und Vorrichtung zur dreidimensionalen Bilderzeugung eines Objektes mit zwei Kreisbahnen gemeinsamer Achse

Method and apparatus for reconstructing three-dimensional images of an object using two common-axis circular paths

(84) Etats contractants désignés:
**DE ES GB IT NL SE**

(30) Priorité: **29.11.1990 FR 9014957**

(43) Date de publication de la demande:
**03.06.1992 Bulletin 1992/23**

(73) Titulaire: **COMMISSARIAT A L'ENERGIE ATOMIQUE**
**75015 Paris (FR)**

(72) Inventeurs:
• **Grangeat, Pierre**
  **F-38330 Saint Ismier (FR)**
• **Le Masson, Patrick**
  **F-38250 Villars de Lans (FR)**

(74) Mandataire: **Ilgart, Jean-Christophe et al**
**c/o Société de Protection des Inventions,**
**25, rue de Ponthieu**
**75008 Paris (FR)**

(56) Documents cités:
**EP-A- 0 292 402          EP-A- 0 379 399**
**GB-A- 2 088 670**

• **IEEE.TRANSACTION ON NUCLEAR SCIENCE vol. NS-25, no. 5, Octobre 1978, M. SCHLINDWEIN: 'Iterative three-dimensional Reconstruction from Twin-Cone Beam Projection'**
• **IEEE.TRANSACTION ON NUCLEAR SCIENCE vol. NS-26, no. 2, Avril 1979, KOWALSKI: 'Multislice Reconstruction from Twin-Cone Scanning'**

## Description

L'invention se rapporte à un procédé et un dispositif de reconstruction d'images tridimensionnelles d'un objet qui comporte des mesures sur deux trajectoires circulaires d'axe commun, c'est-à-dire que les trajectoires appartiennent à des plans parallèles et leurs centres sont joints par un axe perpendiculaire au plan des trajectoires. Si les plans des trajectoires sont confondus, elles sont concentriques. On peut aussi envisager des trajectoires plus nombreuses.

La reconstruction des images concerne un objet déterminé à examiner, et les moyens matériels employés comportent deux réseaux bidimensionnels de capteurs parcourant chacun une des trajectoires ou, de manière équivalente, un seul réseau qui parcourt toutes les trajectoires.

L'image de l'objet est définie par des valeurs prises par une fonction sur chacun de ses points. La fonction est d'autre part une propriété d'un rayonnement (X ou gamma entre autres) de forme conique ayant un point focal et qui passe à travers l'objet ; chaque rayon est reçu par un des capteurs du réseau bidimensionnel et représente donc la somme de la fonction sur tous les points de l'objet appartenant à ce rayon. Un traitement approprié des sommes sur tous les rayons, et ceci pour un nombre suffisant de mesures suivant des incidences différentes autour de l'objet, permet de reconstituer l'image de l'objet.

On se contente en pratique de considérer des nombres finis de rayons et de points de l'objet selon des discrétisations ou des maillages.

Cette invention constitue un perfectionnement d'une invention précédente, décrite dans la demande de brevet européen EP-A-0 292 402, mais on peut envisager d'employer l'invention actuelle dans d'autres circonstances ou en utilisant d'autres méthodes mathématiques de traitement des données.

Les méthodes envisageables utilisent en particulier ce qu'on appelle la transformée de Radon de la fonction à mesurer, qui est définie comme la somme de la fonction sur chacun des plans, appelés plans de Radon, qui passent à travers l'objet considéré ou, mieux encore, la dérivée première de cette transformée. La contribution des points de ces plans qui n'appartiennent pas à l'objet est considérée comme inexistante, ce qui est valable dans le cas d'un rayonnement qui traverse un milieu gazeux ambiant sans être atténué. Ici encore, une discrétisation est effectuée pour n'effectuer les calculs que sur un nombre fini de plans.

La dérivée première de la transformée de Radon est définie comme la dérivée de la transformée de Radon en fonction de la variable $\rho$ définissant la distance du plan considéré à une origine. Pour chaque plan, elle correspond à la somme sur ce plan de la dérivée première de la transformée de Radon dans la direction perpendiculaire au plan. L'invention décrite dans le brevet EP-A-0 292 402 montre que pour un plan passant par une position du point focal du cône de rayonnement et rencontrant le réseau bidimensionnel de capteurs, il est possible de calculer à partir des mesures la valeur exacte de la dérivée première de la transformée de Radon sur ce plan. Ce calcul, suivant les formules exposées dans le brevet EP-A-0 292 402, met en oeuvre de façon préférentielle une pondération de correction d'éloignement du point focal, deux filtrages correspondant aux calculs des dérivées premières respectivement suivant les lignes et les colonnes du réseau de capteurs, deux sommations suivant la droite d'intersection entre le plan à traiter et le réseau bidimensionnel de capteurs, puis une combinaison linéaire et une normalisation des résultats. Cette sommation met en oeuvre les interpolations nécessaires car ces droites d'intersection passent entre les rangées de capteurs ou les coupent.

Dans le reste de la demande, on entend par somme la somme pondérée des valeurs mesurées (dans le cas de la transformée de Radon) aussi bien que la combinaison linéaire des sommes des valeurs pondérées et filtrées (dans le cas de la dérivée première de la transformée de Radon) obtenues selon les lignes et les colonnes du réseau.

La somme de la fonction sur les points des plans de Radon est facile à obtenir à condition que ces plans aient une intersection avec le réseau bidimensionnel de capteurs et qu'ils passent par le point focal unique que visent les capteurs. Il suffit d'effectuer la somme des valeurs mesurées par chaque capteur situé à l'intersection, avec les interpolations nécessaires car les intersections des plans de Radon passent entre des rangées de capteurs ou les coupent. Une fois que les valeurs de la fonction sur les plans de Radon ont été calculées, il existe des formules d'inversion, exposées dans la demande de brevet mentionnée ci-dessus, qui permettent de parvenir aux valeurs de la fonction sur les points du maillage de l'objet correspondant à l'image à reconstruire.

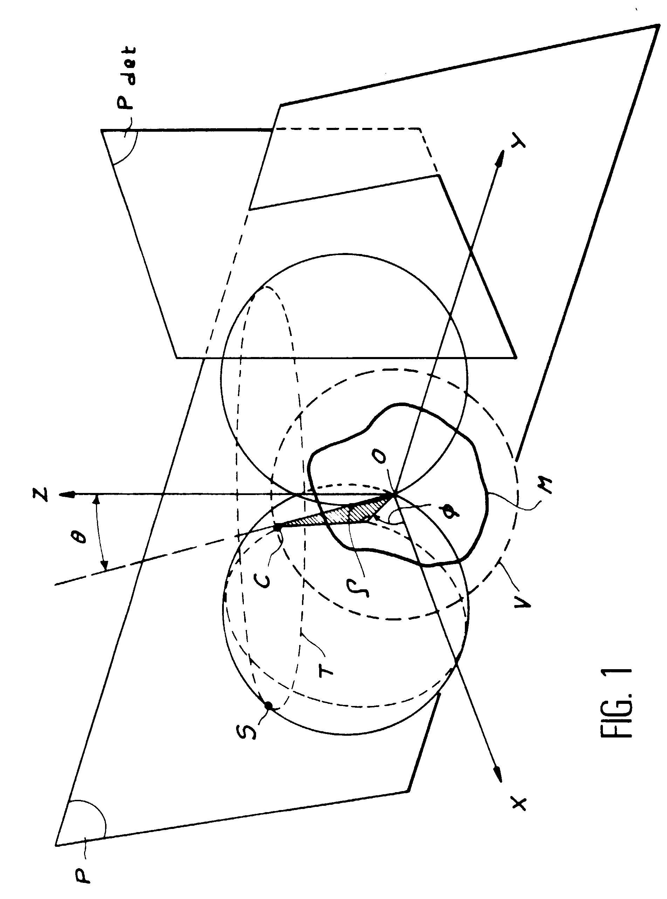

Il faut toutefois revenir sur les conditions qui permettent d'obtenir un nombre de plans de Radon suffisant pour permettre une description satisfaisante de l'objet. Chaque plan de Radon peut être défini par ce qu'on appelle son point caractéristique, c'est-à-dire le point de projection sur ce plan d'une origine O choisie arbitrairement. Ce point caractéristique, noté C sur la figure 1, peut être défini par ses coordonnées sphériques $\rho$, $\phi$ et $\theta$ de rayon, longitude et colatitude respectivement à partir de l'origine O. Le plan de Radon P passant par le point caractéristique C peut être défini par le rayon $\rho$ et le vecteur unitaire $\vec{n}$ de direction $\overrightarrow{OC}$.

Les valeurs de la fonction sur les plans de Radon ne peuvent effectivement être calculées que pour les plans de Radon qui coupent la trajectoire parcourue par le point focal du rayonnement. Ce point focal est concrètement, dans le cas d'une fonction d'atténuation, une source ponctuelle de rayons X, gamma, etc. C'est la conception qui est décrite dans la demande de brevet européenne mentionnée. Les mêmes conditions géométriques existent en tomographie d'émission, quand la fonction à mesurer est l'activité émise par le corps. Le point focal n'a alors pas d'existence physique et correspond simplement au point de convergence vers lequel tous les collimateurs utilisés devant le réseau bidimensionnel sont dirigés.

Il faut, pour obtenir un nombre suffisant de plans de Radon, effectuer plusieurs mesures avec des positions différentes du point focal.

Considérons une trajectoire circulaire T parcourue par le point focal (ou la source) S, le réseau plan de capteurs Pdét parcourant la même trajectoire que le point focal S ou éventuellement une trajectoire concentrique de rayon différent. Supposons encore que l'origine O qui sert à définir les points caractéristiques C soit située sur l'axe de la trajectoire T. Le volume enveloppant les points caractéristiques correspond à un tore To produit par la rotation d'une surface sphérique de diamètre OS autour de l'axe de rotation de la trajectoire T. En effet, les plans de Radon qui passent par le point focal S ont leurs points caractéristiques répartis sur la surface sphérique de diamètre OS car l'angle SCO est droit. Le tore To est appelé le volume caractéristique des mesures, qui dépend donc de la forme de la trajectoire T et de sa position par rapport à l'origine 0.

Il est d'autre part suffisant, pour obtenir une description complète de l'objet par les procédés utilisant les sommes de la fonction sur les plans de Radon, de pouvoir disposer des points caractéristiques appartenant à un volume caractéristique de l'objet. Ce volume caractéristique de l'objet est toujours inclus dans une sphère V centrée sur l'origine O et qui englobe l'objet M. On peut donc assurer que si le volume caractéristique de l'objet est inclus dans le volume caractéristique des mesures, la reconstruction de l'image de l'objet M sera possible. Dans le cas d'une trajectoire circulaire T, cette condition n'est malheureusement pas remplie car une zone d'ombre subsiste pour les points caractéristiques de la sphère V qui n'appartiennent pas au tore To et dont les plans de Radon ne coupent pas la trajectoire T.

L'objet essentiel de l'invention est de faciliter les reconstructions en les rendant plus rapides si plusieurs chaînes de mesure sont employées, ce qui permet de réduire la durée des examens, en imagerie médicale où le patient doit rester immobile et en contrôle non destructif où on est astreint à des cadences de travail ; ou encore, le procédé permet de traiter des cas où chaque chaîne d'acquisition est définie par des paramètres propres et en particulier le cas où les deux centres des trajectoires de deux chaînes d'acquisition sont proches mais distincts pour des raisons mécaniques. C'est alors la qualité des images qui est améliorée. Enfin, quand les deux trajectoires circulaires sont de part et d'autre de l'objet, la zone d'ombre peut être réduite et L'image améliorée ici encore. La superposition de plusieurs trajectoires permet aussi d'augmenter le volume caractéristique de mesures et autorise l'examen de plus gros objets. Mais on se heurte dans tous les cas à des difficultés de calcul car le regroupement des acquisitions faites sur plusieurs trajectoires nécessite des conversions mettant en oeuvre des opérations mathématiques à cause du paramétrage de chaque série de mesures par des coefficients caractéristiques propres.

De plus, si on utilise des chaînes de mesures différentes et si les sommes de la fonction sont d'abord produites dans des repères séparés, les imprécisions mécaniques sur l'orientation réelle des capteurs, les rayons réels de leur trajectoire et du point focal, etc., se traduisent par des calculs et des interpolations supplémentaires quand les changements de repère sont effectués pour regrouper les résultats des séries de mesures. Les informations de reconstruction des images sont donc dégradées. Si par contre on utilise le procédé conforme à l'invention, les paramètres des trajectoires et de séries de mesures, qui intègrent les imprécisions mécaniques et ont été mesurés au cours d'étalonnages préliminaires de la machine, sont utilisés directement dans le repère utilisé pour reconstruire l'objet, dont les images seront alors reconstruites avec une netteté meilleure.

L'invention repose en conséquence sur la constatation que, si on définit un repère commun pour paramétrer les trajectoires et les volumes caractéristiques de mesure, aucune conversion de coordonnées ni de changement de repère n'est nécessaire pour fusionner ou regrouper les résultats provenant de trajectoires différentes. On obtient directement les informations intéressantes dans le repère d'objet dans lequel le maillage utilisé pour reconstruire les images est défini. Or, une telle façon de procéder est facile si les trajectoires sont coaxiales.

L'invention est, pour récapituler, relative à un procédé de reconstruction d'images tridimensionnelles conforme à la revendication 1 et un dispositif conforme à la revendication 6.

Avantageusement, le repère d'objet a un axe confondu avec l'axe perpendiculaire aux trajectoires.

Les coordonnées utilisées sont avantageusement sphériques.

L'invention est aussi relative aux dispositifs conçus spécialement pour la mise en oeuvre du procédé.

Plusieurs variantes de l'invention sont possibles en utilisant des dispositifs légèrement différents et ces procédés vont être décrits en référence aux figures suivantes annexées à titre illustratif et non limitatif :

- la figure 1, déjà décrite, indique les conditions géométriques liées à des acquisitions à l'aide de trajectoires circulaires ;
- la figure 2 représente une généralisation de ces conditions géométriques ;
- la figure 3 représente un mode d'acquisition de mesures ;
- la figure 4 représente un dispositif approprié à l'acquisition des mesures selon la figure 3 ; et
- les figures 5 à 7 représentent trois dispositifs plus complexes envisageables en accord avec l'invention.

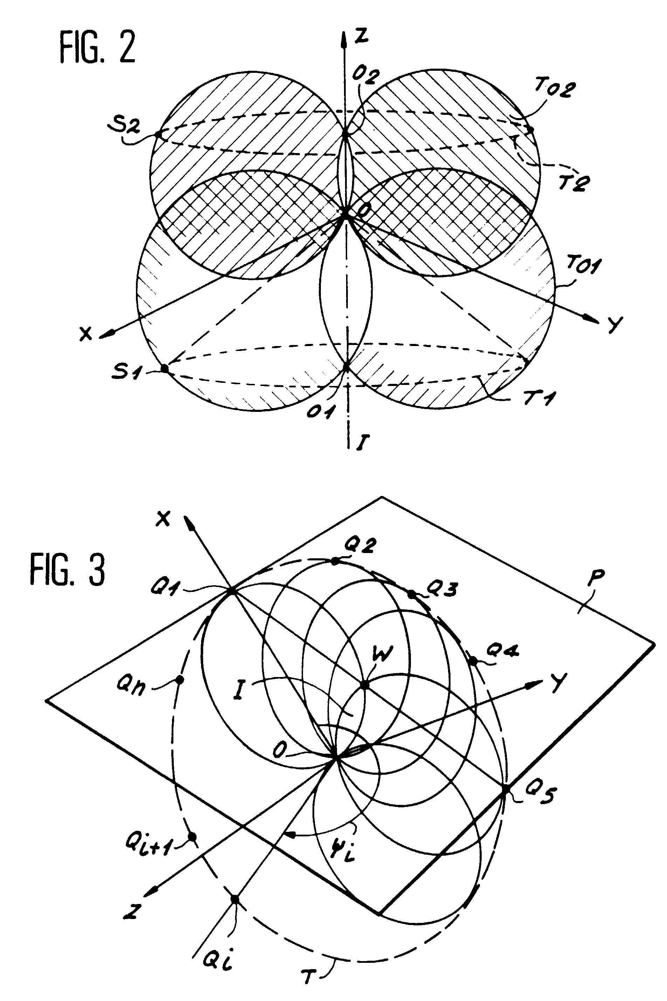

Examinons maintenant le cas de la figure 2, où la trajectoire circulaire référencée par T1 et de centre 01 appartient à un plan qui passe à distance de l'origine O, ce plan étant perpendiculaire à l'axe Z. Le volume caractéristique des mesures, c'est-à-dire l'ensemble des points caractéristiques des plans de Radon définis à partir de l'origine O, est à nouveau un tore To1 engendré en faisant tourner une surface sphérique de diamètre OS1 le long de la trajectoire T1 et autour de l'origine O. Le tore To1 a une forme complexe puisqu'il présente une partie d'intersection I au centre, car les coupes méridiennes du tore To1 forment deux surfaces circulaires qui se recouvrent. Un raisonnement géométrique simple permet de se convaincre que les plans associés aux points situés à l'intérieur de la zone d'intersection I ne coupent pas la trajectoire et que cette partie appartient à la zone d'ombre : le volume caractéristique des mesures représenté suivant cette coupe méridienne est celui qui est hachuré.

On a représenté la superposition d'un autre tore To2 résultant de la rotation d'un cercle de diamètre OS2 autour d'une autre trajectoire T2 de centre O2 distinct de l'origine O, qui est ici représentée sensiblement au centre du segment 0102. Le volume caractéristique de mesures correspondant est également hachuré. On s'aperçoit que bien que ces volumes caractéristiques ne coïncident pas et que la multiplication des trajectoires ayant un axe commun permette de décrire des objets plus volumineux, il reste toujours une zone d'ombre autour de l'axe Z. Le procédé de l'invention n'est donc pas approprié pour décrire parfaitement les objets examinés à cet endroit même si le volume de la zone d'ombre est réduit. En pratique, on effectuera des interpolations à l'intérieur de la zone d'ombre conformément aux indications de l'invention antérieure.

Un mode possible pour la réalisation du procédé a été représenté sur la figure 3. Les deux trajectoires sont confondues en une trajectoire unique T sur laquelle on effectue n mesures en des points régulièrement répartis Q1 à Qn définis par des angles $\psi i = \psi(i-1) + \frac{2\pi}{n}$ à partir de l'axe X. On mesure pour chaque point Qi les valeurs de la fonction sur les plans de Radon dont les points caractéristiques appartiennent à la sphère de diamètre OQi. Dans le cas général, la somme de la fonction sur ces plans peut être calculée à partir de deux positions du point focal car ces plans coupent la trajectoire T en deux points, Q1 et Q5 dans l'exemple considéré. En pratique, les points W retenus sont cependant des points qui sont définis régulièrement sur un maillage défini dans le repère O,X,Y,Z par ses coordonnées sphériques régulières (rayon $\rho$, longitude $\phi$ et colatitude $\theta$), si bien que le plan de Radon ne coupe pas la trajectoire exactement aux points Q et que des approximations ou des interpolations sur les angles $\psi i$ sont donc nécessaires, suivant la méthode envisagée dans l'invention précédente par exemple.

Selon la présente invention, on dispose de deux chaînes de mesures dont chacune effectue les mesures simultanément sur un sous-ensemble des points Q. Les deux façons les plus normales de procéder consistent soit à attribuer les points d'ordre impair à l'un et les points d'ordre pair à l'autre, soit à partager la trajectoire en deux moitiés égales dont les points Q respectifs sont affectés à l'une des chaînes de mesures, chaque sous-ensemble comprenant alors une moitié des points Q. Il est encore possible que les sous-ensembles soient plus grands qu'une moitié, ce qui rendra possibles des recouvrements pour certains points Q. Une autre idée consiste à doubler les mesures aux points Q pour améliorer la qualité de l'image par une moyenne statistique.

Pour chacun des points du volume caractéristique de l'objet, le regroupement des séries de mesures consiste par exemple soit à considérer alternativement les sommes de fonction associées à chaque série de mesures pour les plans rencontrant les deux trajectoires des points focaux, soit à faire leurs moyennes, soit à ne considérer qu'une somme de fonction associée quand le plan ne rencontre qu'une des trajectoires.

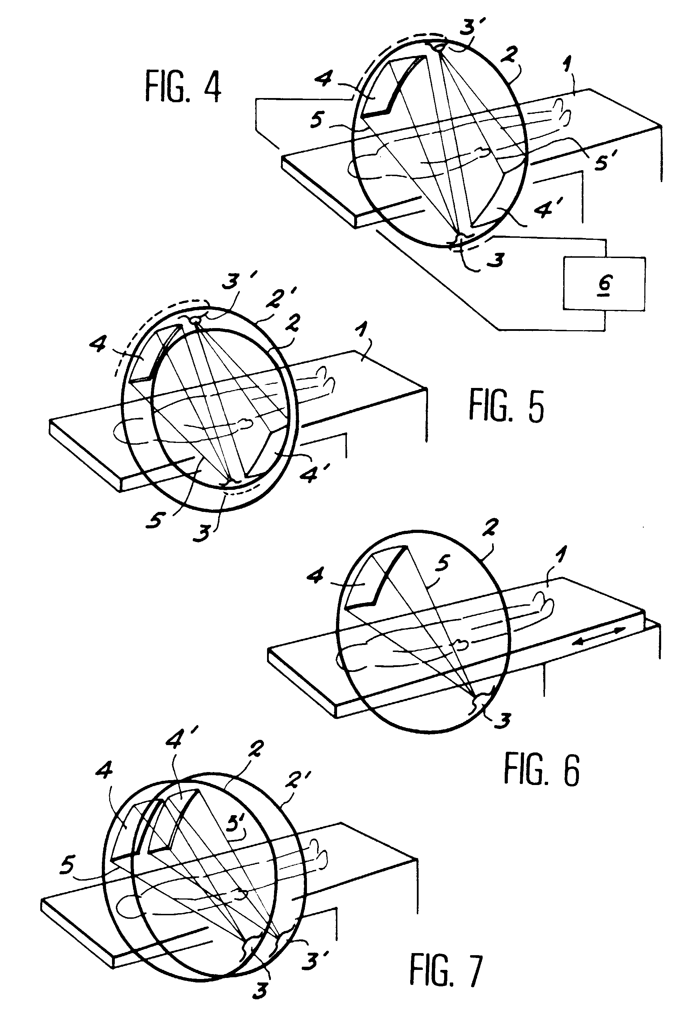

Un dispositif approprié est matérialisé sur la figure 4. Il s'agit d'une installation de tomographie médicale où le patient est couché sur une table 1 horizontale. Un rail circulaire 2 s'étend dans un plan vertical et transversal et entoure la table 1. Il s'agit en fait d'une crémaillère qui porte deux chariots mobiles qui engrènent sur elle. Ces chariots ont été schématisés et on peut se reporter le cas échéant à l'invention antérieure pour plus de détails techniques. Chacun d'eux porte une source de rayons X à proximité d'un écran portant un réseau bidimensionnel de capteurs. Les capteurs de l'écran 4 sont focalisés sur la source 3 de l'autre chariot mobile, et ceux de l'autre écran 4' sont focalisés sur la source 3' du premier chariot mobile. Les sources 3 et 3' émettent un faisceau conique 5 ou 5'. L'objet à examiner est placé dans la zone d'intersection des cônes des deux faisceaux 5 et 5'. En fonctionnement, les deux chariots mobiles se déplacent simultanément en rotation de manière à rester diamétralement opposés. Les mouvements sont réglés par l'ordinateur de commande de l'installation. Dans le cas d'une installation analogue en tomographie d'émission, les sources 3 et 3' seraient omises et le point focal des capteurs de chaque écran 4 ou 4' serait avantageusement situé au centre de l'autre écran 4 ou 4', pour simplifier les calculs d'acquisition. Il serait encore possible de séparer les sources 3, 3' des écrans 4, 4' de sorte que les faisceaux 5 et 5' se coupent à angle droit par exemple. Les écrans 4 et 4' sont reliés à des moyens d'acquisition et de traitement des mesures représentés généralement par 6.

Une conception un peu différente est représentée à la figure 5. Les chariots mobiles sont identiques aux précédents mais placés cette fois-ci sur un rail respectif 2 ou 2'. Les rails 2 et 2' sont coplanaires et concentriques. Rien n'est modifié à l'exception des coefficients des relations géométriques de grossissement en fonction des distances des sources 3 et 3' et des écrans 4 et 4' à l'origine O.

L'invention peut être mise en oeuvre (figure 6) avec un rail unique 2 qui porte une source unique 3 diamétralement opposée à un écran 4 portant un réseau bidimensionnel de capteurs. Dans cette conception, on fait effectuer deux tours complets à la source 3 et l'écran 4, entre lesquels la table 1 subit une translation perpendiculaire au plan du rail 2. On utilise une telle solution pour accroître le volume caractéristique des mesures conformément à l'illustration de la figure 2.

Le même résultat peut être obtenu en dédoublant le dispositif (figure 7) : deux rails circulaires 2 et 2' portent chacun une source 3 ou 3' et un écran 4 ou 4'. Les déplacements sur les deux rails 2 et 2' sont simultanés, ce qui accélère l'acquisition des mesures.

Si les résultats pour un plan de Radon déterminé sont obtenus à chaque trajectoire, une moyenne ou une imbrication sont effectuées avant de procéder aux opérations de reconstruction de l'image, telles que les opérations de filtrage, de pondération et de rétroprojection décrites dans le brevet antérieur.

L'invention peut être mise en oeuvre d'autres manières. C'est ainsi que le débattement angulaire sur chaque trajectoire circulaire peut être réduit à une partie de circonférence. Un cas avantageux est celui d'une circonférence complète formée par l'union des débattements angulaires.

On rappelle en particulier que des trajectoires plus nombreuses sont possibles sans sortir du cadre de l'invention.

Par ailleurs, on a considéré avant tout l'utilisation de ce procédé pour l'inversion de la transformée de Radon proprement dite ou de sa dérivée première. On peut encore procéder sensiolement de la même façon avec une transformée de Fourier de tous les rayons de l'espace de Radon qui ne coupent pas la zone d'ombre, et ceci pour chacun des volumes caractéristiques de mesures. Le regroupement des mesures ainsi transformées peut être mené dans ce repère commun.

Au lieu de la transformée de Radon ou de sa dérivée première, on peut encore procéder de la même façon avec la transformée de Hilbert de ces transformées.

L'invention peut servir pour les applications usuelles d'imagerie médicale ou de contrôle non destructif de pièces.

**Revendications**

1. Procédé de reconstruction d'images tridimensionnelles d'un objet (M) défini par des valeurs prises par une fonction sur des points de l'objet, la fonction étant une propriété de l'objet exprimée par un rayonnement conique (5, 5') émis par l'objet ou atténué par l'objet, ayant un point focal et passant à travers l'objet (M), dans lequel la fonction est calculée par un algorithme d'inversion utilisant des sommes de la fonction calculées sur des groupes desdits points appartenant à des plans (P) passant par au moins un point de l'objet et le point focal et définis par des coordonnées exprimées dans un repère d'objet ; le procédé comportant au moins deux séries de mesures, chaque somme de la fonction sur lesdits plans de l'objet étant calculée à partir d'au moins une des séries de mesures, chacune des séries de mesures étant menée avec un réseau bidimensionnel de capteurs de rayonnement, orientés vers le point focal, et qui est déplacé autour de l'objet sur une trajectoire circulaire respective ; procédé caractérisé en ce que les trajectoires ont des centres (01, 02) joints par un axe perpendiculaire aux trajectoires, sont définies de même que l'objet par rapport au repère d'objet, en ce que, pour chacune des séries de mesures, une série des sommes de la fonction est calculée dans le repère d'objet pour certains des plans de l'objet, qui sont sécants ou tangents à la trajectoire du point focal pendant la série de mesures, et en ce que les séries de sommes de la fonction sont regroupées en une série unique avant d'appliquer l'algorithme d'inversion.

2. Procédé de reconstruction d'images tridimensionnelles suivant la revendication 1, caractérisé en ce que le repère d'objet a un axe (Z) confondu avec l'axe perpendiculaire aux trajectoires.

3. Procédé de reconstruction d'images tridimensionnelles suivant l'une quelconque des revendications 1 ou 2, caractérisé en ce que les coordonnées sont des coordonnées sphériques.

4. Procédé de reconstruction d'images tridimensionnelles suivant l'une quelconque des revendications 1 à 3, caractérisé en ce que le rayonnement est un rayonnement d'atténuation émis par une source ponctuelle au point focal (S), tournant autour de l'objet suivant une trajectoire circulaire de source.

5. Procédé de reconstruction d'images tridimensionnelles suivant l'une quelconque des revendications 1 à 3, caractérisé en ce que le rayonnement est un rayonnement d'émission provenant de l'objet, le point focal étant un point vers lequel convergent les directions de mesure des capteurs.

6. Dispositif de reconstruction d'images tridimensionnelles comprenant au moins un réseau bidimensionnel de capteurs, le réseau étant déplacé autour d'un objet à examiner suivant au moins deux trajectoires circulaires et relié à des moyens d'acquisition de mesures et de traitement des mesures appropriés à la mise en oeuvre d'un procédé conforme à l'une quelconque des revendications précédentes.

**Patentansprüche**

1. Verfahren zur dreidimensionalen Bilderzeugung eines Objekts (M), definiert durch Werte, die mittels einer Funktion an Punkten des Objekts gemessen werden, wobei die Funktion eine Eigenschaft des Objekts ist, ausgedrückt durch eine konische Strahlung (5, 5'), abgestrahlt durch das Objekt oder gedämpft durch das Objekt, mit einem Fokalpunkt und das Objekt (M) durchquerend, bei dem die Funktion durch einen Inversionsalgorithmus berechnet wird, indem Summen der Funktion benutzt werden, berechnet aufgrund von Gruppen der genannten Punkte, die zu Ebenen (P) gehören, die wenigstens einen Punkt des Objekts und den Fokalpunkt durchlaufen, definiert durch in einem Objektbezugspunkt ausgedrückte Koordinaten; wobei das Verfahren wenigstens zwei Meßreihen umfaßt, jede Summe der Funktion auf den genannten Ebenen des Objekts aufgrund wenigstens einer der Meßreihen berechnet wird, jede der Meßreihen mit einem zweidimensionalen Strahlungssensorengitter durchgeführt wird, ausgerichtet auf den Fokalpunkt, der auf einer jeweils kreisförmigen Bahn um das Objekt herum bewegt wird,
**dadurch gekennzeichnet,**
daß die Bahnen Mittelpunkte (01, 02) haben, verbunden durch eine zu den Bahnen senkrechte Achse, ebenso wie das Objekt in bezug auf den Objektbezugspunkt definiert werden, dadurch, daß für jede der Meßreihen eine Summenreihe der Funktion in dem Objektbezugspunkt für bestimmte Ebenen des Objekts berechnet wird, die die Bahn des Fokalpunkts während der Meßreihe schneiden oder tangieren, und dadurch, daß die Summenreihen der Funktion vor dem Anwenden des Inversionsalgorithmus zu einer einzigen Reihe zusammengefaßt werden.

2. Verfahren zur dreidimensionalen Bilderzeugung nach Anspruch 1, dadurch gekennzeichnet, daß der Objektbezugspunkt eine Achse (Z) hat, die mit der zur Bahn senkrechten Achse zusammenfällt.

3. Verfahren zur dreidimensionalen Bilderzeugung nach einem der Ansprüche 1 oder 2, dadurch gekennzeichnet, daß die Koordinaten Kugelkoordinaten sind.

4. Verfahren zur dreidimensionalen Bilderzeugung nach einem der Ansprüche 1 bis 3, dadurch gekennzeichnet, daß die Strahlung eine Schwächungs- bzw. Dämpfungsstrahlung ist, abgestrahlt von einer punktförmigen Quelle mit dem Fokalpunkt (S), die das Objekt entsprechend einer kreisförmigen Quellenbahn umläuft.

5. Verfahren zur dreidimensionalen Bilderzeugung nach einem der Ansprüche 1 bis 3, dadurch gekennzeichnet, daß die Strahlung eine von einem Objekt stammende Emissionsstrahlung ist, wobei der Fokalpunkt ein Punkt ist, gegen den die Meßrichtungen der Sensoren konvergieren.

6. Vorrichtung zur dreidimensionalen Bilderzeugung mit wenigstens einem zweidimensionalen Sensorengitter, wobei das Gitter auf wenigstens zwei kreisförmigen Bahnen um ein zu untersuchendes Objekt herum bewegt wird und mit Erfassungs- und Verarbeitungs-einrichtungen der Messungen verbunden ist, die geeignet sind für die Durchführung eines Verfahrens nach einem der vorangehenden Ansprüche.

**Claims**

1. Process for the reconstruction of three-dimensional images of an object (M) defined by values assumed by a function on points of the object, the function being a property of the object expressed by a conical radiation (5, 5') emitted by or attenuated by the object, having a focal point and passing through the object (M), in which the function is calculated by an inversion algorithm using sums of the function calculated on groups of said points belonging to planes (P) passing through at least one point of the object and the focal point and defined by coordinates expressed in an object reference frame, the process involving at least two series of measurements, each sum of the function on said planes of the object being calculated from at least one of the series of measurements, each of the series of measurements being performed with a bidimensional array of radiation sensors oriented towards the focal point and which is displaced about the object on a respective circular path, said process being characterized in that the paths have centres (01, 02) joined by an axis perpendicular to the paths, are defined in the same way as the object with respect to the object reference frame, in that for each of the series of measurements, a series of sums of the functions is calculated in the object reference frame for certain of the planes of the object, which are secant or tangential to the path of the focal point during the series of measurements and in that the series of sums of the function are pooled in a single series prior to the application of the inversion algorithm.

2. Process for the reconstruction of three-dimensional images according to claim 1, characterized in that the object reference frame has an axis (Z) coinciding with the axis perpendicular to the paths.

3. Process for the reconstruction of three-dimensional images according to claims 1 or 2, characterized in that the coordinates are spherical coordinates.

4. Process for the reconstruction of three-dimensional images according to any one of the claims 1 to 3, characterized in that the radiation is an attenuation radiation emitted by a point source at the focal point (S) rotating about the object in accordance with a circular source path.

5. Process for the reconstruction of three-dimensional images according to any one of the claims 1 to 3, characterized in that the radiation is an emission radiation coming from the object, the focal point being a point towards which the measurement directions of the sensors converge.

6. Apparatus for the reconstruction of three-dimensional images comprising at least one bidimensional array of sensors, the array being displaced about an object to be examined in accordance with at least two circular paths and connected to means for the acquisition and processing of measurements appropriate for the performance of a process according to any one of the preceding claims.

FIG. 1

FIG. 2

FIG. 3

FIG. 4

FIG. 5

FIG. 6

FIG. 7